**Europäisches Patentamt**

(19) **European Patent Office**

**Office européen des brevets**

(11) Publication number : **0 336 454 B1**

## (12) EUROPEAN PATENT SPECIFICATION

(45) Date of publication of patent specification :
**16.09.92 Bulletin 92/38**

(51) Int. Cl.⁵ : **G01N 33/543,** G01N 33/53,
C12Q 1/68

(21) Application number : **89109006.0**

(22) Date of filing : **18.05.85**

(54) **Nucleic acid hybridization assay.**

(30) Priority : **01.06.84 US 616132**
**01.03.85 US 707420**

(43) Date of publication of application :
**11.10.89 Bulletin 89/41**

(45) Publication of the grant of the patent :
**16.09.92 Bulletin 92/38**

(84) Designated Contracting States :
**AT BE CH DE FR GB IT LI LU NL SE**

(56) References cited :
EP-A- 0 133 671
EP-A- 0 139 489
EP-A- 0 146 039
EP-A- 0 146 815
BIOCHEMISTRY, vol. 17, no. 26, December
1978, American Chemical Society;
W.E.STUMPH et al., pp. 5791-5798

(56) References cited :
CHEMICAL ABSTRACTS, vol. 70, no. 25, June
1969, Columbus, OH (US); E.F.SCHWARTZ et
al., p. 168, no. 113344d
CHEMICAL ABSTRACTS, vol. 98, no. 1,
January 1983, Columbus, OH (US); A.C.VAN
PROOIJEN-KNEGT et al., p. 112, no. 1157s

(60) Publication number of the earlier application in
accordance with Art. 76 EPC : **0 163 220**

(73) Proprietor : **MILES INC.**
**1127 Myrtle Street**
**Elkhart Indiana 46514 (US)**

(72) Inventor : **Carrico, Robert J.**
**54256 Silver Street**
**Elkhart, IN 46514 (US)**

(74) Representative : **Dänner, Klaus, Dr. et al**
**c/o Bayer AG Konzernverwaltung RP**
**Patentabteilung**
**W-5090 Leverkusen 1 Bayerwerk (DE)**

## Description

This invention relates to nucleic acid hybridization assay methods and reagent systems for detecting specific polynucleotide sequences. The principle of nucleic acid hybridization assays was developed by workers in the recombinant DNA field as a means for determining and isolating particular polynucleotide base sequences of,interest. It was found that single stranded nucleic acids, e.g., DNA and RNA, such as obtained by denaturing their double stranded forms, will hybridize or recombine under appropriate conditions with complementary single stranded nucleic acids. By labeling such complementary probe nucleic acids with some readily detectable chemical group, it was then made possible to detect the presence of any polynucleotide sequence of interest in a test medium containing sample nucleic acids in single stranded form.

In addition to the recombinant DNA field, the analytical hybridization technique can be applied to the detection of polynucleotides of importance in the fields of human and veterinary medicine, agriculture, and food science, among others. In particular, the technique can be used to detect and identify etiological agents such as bacteria and viruses, to screen bacteria for antibiotic resistance, to aid in the diagnosis of genetic disorders such as sickle cell anemia and thalassemia, and to detect cancerous cells. A general review of the technique and its present and future significance is provided in Biotechnology (August 1983), pp. 471-478.

## BACKGROUND INFORMATION

The following information is provided for the purpose of making known information believed by the applicant to be of possible relevance to the present invention. No admission is necessarily intended, nor should be construed, that any of the following information constitutes prior art against the present invention.

The state-of-the-art nucleic acid hybridization assay techniques generally involve immobilization of the sample nucleic acid on a solid support. Hybridization between particular base sequences or genes of interest in the sample nucleic acid is determined by separating the solid support from the remainder of the reaction mixture which contains unbound labeled probe, followed by detection of the label on the solid support.

The need to immobilize sample nucleic acids in order to conduct the state-of-the-art hybridization assay poses two significant problems. Firstly, the procedures required to accomplish immobilization are generally time consuming and add a step which is undesirable for routine use of the technique in a clinical laboratory. Secondly, proteins and other materials in the heterogeneous sample, particularly in the case of clinical samples, can interfere with the immobilization of the nucleic acids.

As alternatives to immobilizing sample nucleic acids and adding labeled probe, one can use an immobilized probe and label the sample nucleic acids in situ, or one can use a dual hybridization technique requiring two probes, one of which is immobilized and the other labeled [Methods in Enzymology 65:468(1968) and Gene 21:77-86(1983)]. The former alternative, however, is even less desirable since the in situ labeling of the sample nucleic acids requires a high degree of technical skill which is not routinely found in clinical technicians and there are no simple, reliable methods for monitoring the labeling yield, which can be a significant problem if the labeling media contain variable amounts of inhibitors of the labeling reaction. The dual hybridization technique has the disadvantages of requiring an additional reagent and incubation step and the kinetics of the hybridization reaction can be slow and inefficient. The accuracy of the assay can also be variable if the complementarity of the two probes with the sample sequence is variable.

Techniques for directly detecting the polynucleotide duplex formed as the product of hybridization between the sample and probe polynucleotides, and thereby dispensing with the chemical labeling and immobilization of sample or probe polynucleotides, have been generally unsatisfactory. Attempts to generate antibodies which will selectively bind double stranded DNA·DNA hybrids over single stranded DNA have failed [Parker and Halloran, "Nucleic Acids in Immunology", ed. Plescia and Braun, Springer-Verlag, NY (1969) pp. 18 et seq]. Some success has been achieved in generating antibodies that will bind DNA·RNA mixed hybrids or RNA·RNA hybrids and have low affinity for the single stranded polynucleotides [see, for example, Rudkin and Stollar, Nature 265:472(1977)]. Rudkin and Stollar fixed whole cells on microscope slides and exposed the DNA in the nucleus. It was hybridized with an RNA probe and the hybrid was detected by fluorescence microscopy with fluorescein-labeled antibody to DNA·RNA. However, these methods are described, as in the case of the hybridization techniques discussed above employing labeled probes, as requiring immobilization of the sample nucleic acids. Immobilizaticn of cellular DNA for in situ hybridization is particularly tenuous because the DNA must remain fixed to delicate cell residues during the hybridization and immunochemical detection steps. The results observed by fluorescence microscopy do not give quantitative data on the amount of hybrid formed. Specific antibodies to double-strand RNA and DNA-RNA hybrids are known from E.F. Schwartz et al.: "Antibodies to polydenylate-polyuridylate copolymers as reagents for duble-strand RNA and DNA-RNA hybrid complexes", in BIOCHEM. BIOPHYS. RES. COMMUN. 1969, 35(1), 115 - 20.

These antibodies have been used to localize DNA-RNA hybrids on chromosomes (see PROOIJEN-KNEGT et al.: "In situ hybridization of DNA sequences in human metaphase chromosones visualized by an indirect fluorescent immunocytochemical procedure ", in EXP. CELL RES. 1982, 141(2), 397-407 and BIOCHEMISTRY, vol. 17, no 26, 26th December 1978, pages 5791 - 98, American Chemical Society, US; W.E. STUMPF et al.: " Gene enrichment using antibodies to DNA-RNA hybrids: Purification and mapping of dictyostelium discoideum rDNA").

The antibodies have not been used in hybridization assays.

Accordingly, there is an established need for a nucleic acid hybridization assay which does not require the immobilization or labeling of sample nucleic acids, and which does not require dual probes. Further, such technique should allow the use of a variety of labels, particularly of the nonradioisotopic type. A nucleic acid hybridization assay method and reagent system having these and other advantages are principal objectives of the present invention.

## SUMMARY OF THE INVENTION

A nucleic acid hybridization assay method has now been devised which eliminates the need to immobilize or label sample nucleic acids and which requires but a single probe element. The present invention provides a method for determining a particular polynucleotide sequence in an appropriate test medium containing single stranded nucleic acids. The test medium is combined with an immobilizable polynucleotide probe, comprising at least one single stranded base sequence which is substantially complementary to the sequence to be determined, under conditions favorable to hybridization between the sequence to be determined and the complementary probe sequence. The complementary probe sequence will be selected to be substantially composed of RNA when the sequence to be determined is RNA or DNA, that is, such probe sequence can be selected to be RNA whether the sample sequence of interest is RNA or DNA. Alternatively, when the sample sequence of interest is RNA, the complementary probe sequence can be selected to be substantially composed of either DNA or RNA. Thus, hybrids resulting from hybridization between the probe and the sample sequence will be DNA·RNA ord RNA·RNA duplexes.

The resulting hybrids can then be detected, after immobilization of the probe as this was combined with the test medium in an immobilizable form, by addition of an antibody reagent capable of binding to the DNA·RNA or RNA·RNA duplexes formed and determining the antibody reagent that becomes bound to such duplexes. A variety of protocols and reagent combinations can be employed in order to carry out the principles of the present method. Important features of the present invention are that the sample nucleic acids are not immobilized or required to be labeled before contact with the probe.

The antibody reagent and its use in the present invention is fully described in parent application EP 163 220.

By eliminating the need to immobilize or label the sample nucleic acids, the present invention provides a highly advantageous hybridization assay technique. The analyst is not required to have the high level of skill or to take the requisite time to perform the immobilization or labeling procedures. Moreover, there is complete elimination of the potential for sample interferences with the immobilization procedure. The test kit provided to the clinical user would include the probe a readily immobilizable form such as by binding to an immobilized binding partner. In the prior art systems, interferences from extraneous proteins and other materials in the sample can be a serious problem whether the sample nucleic acids to be immobilized are RNA or DNA.

In the prior art methods, immobilization is accomplished by adsorption onto a microporous membrane, such as nitrocellulose, or by covalent bonding to reactive sites on a solid support. In the first case, proteins from the sample can coat the surface and block the adsorption of nucleic acids. Furthermore, many procedures require baking at elevated temperatures, commonly higher than 80°C, in vacuo to fix adsorbed nucleic acids to the support. If mucus or other materials endogeneous to the sample are present, they can become dried to the support to form a film that can adsorb the labeled probe during hybridization and increase the background signal and consequently decrease sensitivity. Also, if an enzyme or other protein is involved in the detection of the label, it can often bind nonspecifically to the film and contribute even further to the backgound problem. If covalent immobilization is employed, proteins and other materials from the sample can be expected to have available reactive groups which will engage in the coupling reaction and neutralize the coupling of the desired nucleic acids.

Since the present invention provides the probe in a form which is readily immobilized by binding to an immobilized binding partner, the inefficiencies inherent in the prior art immobilization procedures are overcome and thus the detection limits of the assay are maintained. A further advantage is that nonspecific binding of sample RNA or DNA to the solid support will not be recognized by the antibody reagent. Therefore, the background signal will be low and the detection limit accordingly improved. Relative to the dual hybridization method which uses both a labeled probe and an immobilized probe, the labeled nucleotide can bind nonspecifically to the solid support and contribute background signal. this is not a possibility in the present method since no labeled probe is involved.

BRIEF DESCRIPTION OF THE DRAWINGS

The drawing is a schematic representation of the preferred method for performing the present invention. The use of nucleic acid hybridization as an analytical tool is based fundamentally on the double stranded, duplex structure of DNA. The hydrogen bonds between the purine and pyrimidine bases of the respective strands in double stranded DNA can be reversibly broken. The two complementary single strands of DNA resulting from this melting or denaturation of DNA will associate (also referred to as reannealing or hybridization) to reform the duplexed structure. As is now well known in the art, contact of a first single stranded nucleic acid, either DNA or RNA, which comprises a base sequence sufficiently complementary to a second single stranded nucleic acid under appropriate solution conditions, will result in the formation of DNA·DNA, DNA·RNA, or RNA·RNA hybrids, as the case may be.

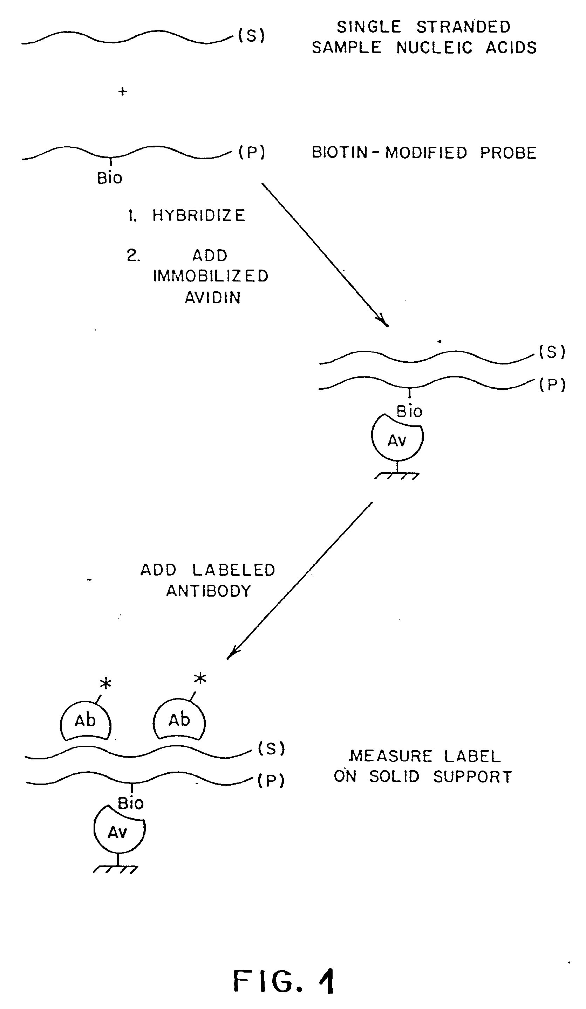

In the embodiment depicted in Fig. 1 of the drawing, the single stranded sample nucleic acids are contacted with a soluble form of the probe which has been appropriately chemically modified to comprise bindable biotin moieties. To the resulting soluble hybrids that are formed is added an immobilized form of avidin, a binding partner for biotin, resulting in formation of immobilized hybrids. The thus immobilized duplexes, optionally after separating them from the remainder of the reaction mixture, are contacted with labeled anti-hybrid antibodies, and after washing, the label present on the solid support is mesured as above.

DESCRIPTION OF THE PREFERRED EMBODIMENTS

*The Probe*

The probe will comprise at least one single stranded base sequence substantially complementary to the sequence to be detected. However, such base sequence need not be a single continuous polynucleotide segment, but can be comprised of two or more individual segments interrupted by noncomplementary sequences. These nonhybridizable sequences can be linear, or they can be self-complementary and form hairpin loops. In addition, the complementary region of the probe can be flanked at the 3'- and 5'-termini by nonhybridizable sequences, such as those comprising the DNA or RNA of a vector into which the complementary sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with sample DNA or RNA, and provided that the antibody reagent selected for use with the probe does not significantly crossreact with the double stranded regions in the probe (e.g., where the antibody reagent is specific for DNA·RNA hybrids and the probe comprises RNA·RNA double stranded regions, or vice versa). The complementary probe sequence can be of any convenient or desired length, ranging from as few as a dozen to as many as 10,000 bases, and including oligonucleotides having less that about 50 bases.

The RNA or DNA probe can be obtained in a variety of conventional manners. For example, in the case of RNA probes, RNA can be isolated as the natural products of cells, such as 5s, l6s and 23s ribosomal RNAs from bacteria or cellular transfer RNAs. It is also practical to isolate specific messenger RNAs from cells which specialize in production of large amounts of a protein for which the messenger codes.

In vitro synthesis of RNA probes can be accomplished with a vector which contains the very active Salmonella typhimurium bacteriophage SP6 transcription promoter [Green et al (1983) Cell 32:681]. A vector with multiple restriction endonuclease sites adjacent to the promoter is available from Promega Biotec, Madison, WI. A DNA probe is cloned into the vector which is then propagated in a bacterial host. Multiple RNA copies of the cloned DNA probe can be synthesized in vitro using DNA dependent RNA polymerase from bacteriophage SP6.

DNA probes can be prepared from a variety of sources. An entire bacterial genome can be immobilized for a hybridization assay designed to detect bacteria in a typically sterile sample. The assay would be capable of detecting abundant bacterial RNAs such as ribosimal RNAS and transfer RNAs. Alternatively, specific DNA sequences complementary to cellular RNAs can be cloned into well known plasmid or viral vectors and used as hybridization probes.

It should be understood that in using the expressions "RNA probe" and "DNA probe" herein, it is not implied that all nucleotides comprised in the probe be ribonucleotides or 2'-deoxyribonucleotides. The fundamental feature of an RNA or DNA probe for purposes of the present invention is that it be of such character to enable the stimulation of antibodies to DNA·RNA or RNA·RNA hybrids comprising an RNA or DNA probe which do not crossreact to an analytically significant degree with the individual single strands forming such hybrids. Therefore, one or more of the 2'-positions on the nucleotides comprised in the probe can be chemically modified provided the antibody binding characteristics necessary for performance of the present assay are maintained to a substantial degree. Likewise, in addition or alternatively to such limited 2'-deoxy modification, a probe can have in general any other modification along its ribose phosphate backbone provided there is no substantial interference with the specificity of the antibody to the double stranded hybridization product compared to its individual single strands.

Where such modifications exist in an RNA or DNA probe, the immunogen used to raise the antibody reagent would preferably comprise one strand having substantially corresponding modifications and the other strand being substantially unmodified RNA of DNA, depending on whether sample RNA or DNA was intended to be detected. Preferably, the modified strand in the immunogen would be identical to the modified strand in an RNA or DNA probe. An example of an immunogen is the hybrid poly(2'-0-methyladenylic acid)·poly(2'-deoxythymidylic acid). Another would be poly (2'-0-ethylinosinic acid)·poly(ribocytidylic acid). The following are further examples of modified nucleotides which could be comprised in a modified probe: 2'-0-methylribonucleotice, 2'-0-ethylribonucleotide, 2'-azidodeoxyribonucleotide, 2'-chlorodeoxyribonucleotide, 2'-0-acetylribonucleotide, and the phosphorothiolates or methylphosphonates of ribonucleotides or deoxyribonucleotides. Modified nucleotides can appear in probes as a result of introduction during enzymic synthesis of the probe from a template. For example, adenosine 5'-0-(I-thiotriphosphate) (ATPαS) and dATPαS are substrates for DNA dependent RNA polymerases and DNA polymerases, respectively. Alternatively, the chemical modification can be introduced after the probe has been prepared. For example, an RNA probe can be 2'-0-acetylated with acetic anhydride under mild conditions in an aqueous solvent [Steward, D. L. et al, (1972) Biochim. Biophys. Acta 262:227].

The critical property of an RNA or DNA probe for use herein is that antibodies raised against the probe duplexed with a complementary RNA or DNA strand, as desired, will discriminate in their binding properties between the duplexed form of the piobe and single stranded nucleic acids. It is this property which enables detection of hybridized probe in the assay mixture without significant background binding to the unhybridized single stranded form of the probe or any nonspecifically bound single stranded sample nucleic acids. While as described above certain modifications along the ribonucleotide or deoxyribonucleotide strand can be tolerated without loss of antibody discrimination of the duplex from single strands, it will generally be preferable to employ RNA probes which are composed entirely of ribonucleotides when the sample polynucleotide is RNA or DNA. DNA probes can be used advantageously when the sample is RNA.

*Immobilization of the Probe*

As described previously, the probe will be presented for hybridization with sample nucleic acids in an immobilizable form. An immobilizable form of the probe will be one in which the probe can be conveniently rendered immobilized subsequent to the hybridization reaction. The means by which the probe is ultimately immobilized is not critical to the present invention and any available approach can be taken so long as hybrids formed between the probe and the sequence of interest are rendered immobilized through a property of the probe. Thus, sample nucleic acids are not subjected to direct immobilization.

The probe can also be presented to the hybridization reaction in an immobilized form, whereby the probe can be in any appropriate form that enables the probe, and any components of the reaction mixture that have become associated therewith by hybridization and/or by binding of the anti-hybrid reagent, to be subsequently isolated or separated from the remaining mixture such as by centrifugation, filtration, chromatography, or decanting.

This is however subject matter which is claimed in corresponding EP 339 686.

A particularly attractive alternative to employing directly immobilized probe is to use an immobilizable form to probe according to the present invention, which allows hybridization to proceed in solution where the kinetics are more rapid. Normally in such embodiment, one would use a probe which comprises a reactive site capable of forming a stable covalent or noncovalent bond with a reaction partner and obtain immobilization by exposure to an immobilized form of such reaction partner. Preferably, such reactive site in the probe is a binding site such as a biotin or hapten moiety which is capable of specific noncovalent binding with a binding substance such as avidin or an antibody which serves as the reaction partner.

Essentially any pair of substances can comprise the reactive site/reactive partner pair which exhibit an appropriate affinity for interacting to form a stable bond, that is a linking or coupling between the two which remains substantially intact during the subsequent assay steps, principally the separation and detection steps. The bond formed may be a covalent bond or a noncovalent interaction, the latter being preferred especially when characterized by a degree of selectivity or specificity. In the case of such preferred bond formation, the reactive site on the probe will be referred to as a binding site and the reaction partner as a binding substance with which it forms a noncovalent, commonly specific, bond or linkage.

In such preferred embodiment, the binding site can be present in a single stranded hybridizable portion or in a single or double stranded nonhybridizable portion of the probe or can be present as a result of a chemical modification of the probe. Examples of binding sites existing in the nucleotide sequence are where the probe comprises a promoter sequence (e.g., lac-promoter, trp-promoter) which is bindable by a promoter protein (e.g, bacteriophage promoters, RNA polymerase), or comprises an operator sequence (e.g., lac operator) which is bindable by a repressor protein (e.g., lac repressor), or comprises rare, antigenic nucleotides or sequences (e.g., 5-bromo or 5-iododeoxyuridine, Z-DNA) which are bindable by specific antibodies [see also British Pat. Spec. 2,l25,964]. Binding sites introduced by chemical modification of the polynucleotide comprised in the probe are particularly useful and normally involve linking one member of a specific binding pair to the probe nucleic acid. Useful binding pairs from which to choose include biotin/avidin (including egg white avidin and streptavidin), haptens and antigens/antibodies, carbohydrates/lectins, enzymes/inhibitors, and the like. Where the binding pair consists of a proteinaceous member and a nonproteinaceous member, it will normally be preferred to link the nonproteinaceous member to the probe since the proteinaceous member may be unstable under the denaturing conditions of hybridization of the probe. Preferable systems involve linking the probe with biotin or a hapten and employing immobilized avidin or anti-hapten antibody reagent, respectively.

When the probe is presented for hybridization with the sequence of interest in an immobilizable form, the subsequent steps of immobilization of the formed duplexes through a property of the probe and addition of the anti-hybrid antibody reagent can proceed in any desired order. Immobilization and anti-hybrid addition can be accomplished by simultaneous addition of the involved reagents and materials, or one can precede the other, with or without intervening wash or separation steps, in either order. Where ordered additions are followed, of course one will take into account the concentrations of the added reagents so as not to oversaturate the formed hybrids and inhibit interaction therewith of the second added materials.

Although immobilizable probes which become bound to solid supports by specific binding processes described above are preferred, immobilizable probes can be bound to supports by processes with relatively low specificity. In this case the support would bind the hybridized probe but not the unhybridized form. Then the amount of hybrid would be measured with the antibody reagent. An example of a support of this type is hydroxyapitite which binds DNA·RNA and RNA·RNA duplexes but not the single stranded species [Brenner and Falkow, Adv. in Genet., 16:81(1973)].

Also, a chemically active or activatable group can be introduced into the probe and allowed to react with the solid support following the hybridization. This system would give a covalently immobilized probe and the amount of hybrid coupled to the support can be determined with the antibody reagent.

## Anti-Hybrid Antibody Reagent and Detection Schemes

The antibody reagent and its use in the present invention is described in more detail in parent application EP 163 220.

## Reaction Mixture

The test sample to be assayed can be any medium of interest, and will usually be a liquid sample of medical veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharnygal aspirates. Where the test sample obtained from the patient or other source to be tested contains principally double stranded nucleic acids, such as contained in cells, the sample will be treated to denature the nucleic acids, and if necessary first to release nucleic acids from cells. Denaturation of nucleic acids is preferably accomplished by heating in boiling water or alkali treatment (e.g., 0.l N sodium hydroxide), which if desired, can simultaneously be used to lyse cells. Also, release of nucleic acids can, for example, be obtained by mechanical disruption (freeze/thaw, abrasion, sonication), physical/chemical disruption (detergents such as Triton®, Tween®, sodium dodecylsulfate, alkali treatment, osmotic shock, or heat), or enzymatic lysis (lysozyme, proteinase K, pepsin). The resulting test medium will contain nucleic acids in single stranded form which can then be assayed according to the present hybridization method.

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 2XSSC where 1XSSC = 0.l5M sodium chloride and 0.0l5M sodium citrate, pH 7.0), protein such as bovine serum albumin, Ficoll® (a trademark identifying a copolymer of sucrose and epichlorohydrin sold by Pharmacia Fine Chemicals, Piscataway, NY), polyvinylpyrrolidone, and a denatured foreign DNA such as from calf thymus or salmon sperm). The degree of complementarity between the sample and probe strands required for hybridization to occur depends on the stringency of the conditions. The extent and specificity of hybridization is affected by the following principal conditions:

1. The purity of the nucleic acid preparation.

2. Base composition of the probe - G-C base pairs will exhibit greater thermal stability than A-T or A-U base pairs. Thus, hybridizations involving higher G-C content will be stable at higher temperatures.

3. Length of homologous base sequence - Any short sequence of bases (e.g., less than 6 bases), has a high degree of probability of being present in many nucleic acids. Thus, little or no specificity can be attained in hybridizations involving such short sequences. The present homologous probe sequence will be at least 10 bases, usually 20 bases or more, and preferably greater than 100 bases. From a practical standpoint, the homologous probe sequence will often be between 300-1000 nucleotides.

4. Ionic strength - The rate of reannealing increases as the ionic strength of the incubation solution increases. Thermal stability of hybrids also increases.

5. Incubation temperature - Optimal reannealing occurs at a temperature about 25-30°C below the melting temperature (Tm) for a given duplex. Incubation at temperatures significantly below the optimum allows less related base sequences to hybridize.

6. Nucleic acid concentration and incubation time - Normally, to drive the reaction towards hybridization, one of the hybridizable sample nucleic acid or probe nucleic acid will be present in excess, usually 100 fold excess or greater.

7. Denaturing reagents - The presence of hydrogen bond disrupting agents such as formamide and urea increases the stringency of hybridization.

8. Incubation time - The longer the incubation time, the more complete will be the hybridization.

9. Volume exclusion agents - The presence of these agents, as exemplified by dextran and dextran sulfate, are thought to increase the effective concentrations of the hybridizing elements thereby increasing the rate of resulting hybridization.

Normally, the temperature conditions selected for hybridization will be incompatible with the binding of antibody reagent to formed hybrids and detection of the label response. Accordingly, the antibody reagent binding step and label detection step will proceed after completion of the hybridization step. The reaction mixture will usually be brought to a temperature in the range of from about 3°C to about 40°C and the binding and detection steps then performed. Dilution of the hybridization mixture prior to addition of antibody reagent is desirable when the salt and/or formamide concentrations are high enought to interfere significantly with the antibody binding reaction.

It might be found in a particular assay situation using an RNA probe that the probe is subject to partial degradation by alkaline hydrolysis of the phosphodiester bonds or by the presence of ribonucleases. In the former case, hydrolysis can be controlled by avoiding exposure of the probe to a pH higher than about 10. Ribonucleases can be effectively inhibited by the presence of such substances as sodium dodecylsulfate, aurintricarboxylic acid, ribonucleoside vanadyl complexes, heparin, diethylpyrocarbonate, and proteinaceous inhibitors isolated from mammalian sources.

*Reagent System*

The present invention additionally provides a reagent system, i.e., reagent combination or means, comprising all of the essential elements required to conduct a desired assay method. The reagent system is presented in a commercially packaged form, as a composition or admixture where the compatability of the reagents will allow, in a test device configuration, or more usually as a test kit, i.e., a packaged combination of one or more containers, devices, or the like holding the necessary reagents, and usually including written instructions for the performance of assays. Reagent systems of the present invention include all configurations and compositions for performing the various hybridization formats described herein.

In all cases, the reagent system will comprise (i) a poly-nucleotide probe which comprises a specific binding site and which at least one single stranded base sequence which is substantially complementary to the sequence to be determined, the complementary probe sequence being substantially composed of RNA when the sequence to be determined is RNA or DNA, or being substantially composed of DNA or RNA when the sequence to be determined is RNA and (ii) an immobilized form of a binding partner for said specific binding site comprised in said probe and (iii) an antibody reagent capable of binding to DNA/RNA or RNA/RNA duplexes formed between the sequence to be determined and the complementary probe sequence, characterized in that the probe is in an immobilizable form. A test kit form of the system can additionally include aucillary chemicals such as the components of the hybridization solution and denaturation agents capable of converting double stranded nucleic acids in a test sample into single stranded form. Preferably, there is included a chemical lysing and denaturing agent, e.g., alkali, for treating the sample to release single stranded nucleic acid therefrom.

The present invention will now be illustrated, but is not intended to be limited, by the following example.

EXAMPLE

Hybridization Assay for 23s Ribosomal RNA Using an Immobilizable DNA Probe

The sample RNA is hybridized with a soluble DNA probe with appended biotin residues. Then the hybridized and unhybridized DNA probe are bound to a solid support with immobilized streptavidin. The amount of DNA·RNA on the support is measured by an immunoassay using enzyme-labeled antibody to DNA·RNA.

A. Biotinylated Probe DNA

The M-l3 virus described above in Example 2 with the insert complementary to 23s RNA is propagated in E. coli strain JMl03 and the bacterial cells are harvested for isolation of the replicative form of the viral DNA. This double stranded DNA is purified by cesium chloride-ethidium bromide density gradient centrifugation [Maniatis, et a., supra].

Biotin residues are introduced into this double stranded DNA by nick translation using biotinylated dUTP available from Enzo Biochem. Inc., NY [Langer, P. R. et al (l98l) Proc. Natl Acad. Sci., 78:6633; Leary, J. J. et al (l983) Proc. Natl. Acad. Sci., 80:4045].

Contaminating RNA is degraded by treatment with alkali as described in Example 2. Immediately before use the biotinylated probe is denatured by placing the solution in a boiling water bath for four minutes.

B. Immobilization of streptavidin

Streptavidin (Calbiochem-Behring Corp., La Jolla, CA) is immobilized on Act-Ultrogel® AcA 22 (available from LKB Instruments, Inc., Gaithersburg, MD) which is an acrylamide-agarose support activated with glutaraldehyde [Doley, S. G. et al (l976) FEBS Letters, 65:87]. The immobilization is carried out according to the manufacturer's instructions to give approximately 0.5 µg streptavidin per 10 µl of packed Act-Ultrogel AcA 22.

C. Hybridization assay

Two milliliter aliquots of urines suspected of containing bacterial infection are centrifuged at 3000 x g to sediment the bacteria and the supernatants are decanted. Ninety microliters of hybridization solution described in Example 2 is added to each pellet and 5 µl of the biotinylated probe (at a concentration of 0.5 µg/ml in 20 mM sodium phosphate buffer, pH 7.0, 0.5 mM EDTA) is added. The mixtures are agitated to suspend the pellets (if present) and they are incubated at 55°C for 4.0 hours.

Then 700 µl of 20 mM sodium phosphate buffer, pH 7.4, containing 5.0 mg bovine albumin and 50 µl of the Ultrogel with immobilized streptavidin is added to each mixture to dilute the hybridization solution and immobilize the biotinylated probe. The mixtures are shaken at room temperature for two hours and the liquid is removed from the support.

The amount of DNA·RNA hybrid associated with the Ultrogel support is measured by immunoassay as described in Example 2 for the cellulose support.

For comparison, the unprocessed urines are tested for bacteria by the one microliter loop culture method using MacConkey and blood agar plates. Plates are incubated at 37°C for 36 hours and colonies are counted.

Urines with high levels of bacteria by the culture method give high absorbances by the hybridization assay.

**Claims**

1. A method for determining a particular polynucleotide sequence in a test medium containing single stranded nucleic acids, comprising the steps of combining the test medium with a probe comprising at least one single stranded base sequence which is substantially complementary to the sequence to be determined under conditions favorable to hybridization between the sequence to be determined and the complementary probe sequence, the complementary probe sequence being substantially composed of RNA when the sequence to be determined is RNA or DNA, or being substantially composed of DNA or RNA when the sequence to be determined is RNA, and detecting hybridized probe by binding of an antibody reagent capable of binding to DNA/RNA or RNA/RNA duplexes formed between the sequence to be determined and the complementary probe sequence and determining the antibody reagent that becomes bound to such duplexes, characterized in that the probe comprises a specific binding site, and before performing said detecting of hybridized probe, an immobilized form of a binding partner for such specific binding site is contacted with probe in order to render said probe immobilized.

2. The method of claim 1 wherein the probe is in a soluble form when contacted with the test medium and is immobilizable when hybridized to the sequence to be determined by contact with an adsorbent which adsorbs double stranded nucleic acids.

3. The method of any one of claims 1 and 2 wherein the antibody reagent is labeled with a detectable chemical group selected from an enzymatically active group, a fluorescer, a chromophere, a luminescer, a specifically bindable ligand, or a radioisotope.

4. The method of claim 1 wherein the test medium comprises a biological sample which has been subjected to conditions to release and denature nucleic acids present therein.

5. A reagent system for detecting a particular polynucleotide sequence in a test medium by nucleic acid hybridization, comprising (i) a polynucleotide probe which comprises a specific binding site and which at least one single stranded base sequence which is substantially complementary to the sequence to be determined, the complementary probe sequence being substantially composed of RNA when the sequence to be determined is RNA or DNA, or being substantially composed of DNA or RNA when the sequence to be determined is RNA and (ii) an immobilized form of a binding partner for said specific binding site comprised in said probe, and (iii) an antibody reagent capable of binding to DNA/RNA or RNA/RNA duplexes formed between the sequence to be determined and the complementary probe sequence, characterized in that the probe is in an immobilizable form.

6. The reagent system of claim 6 or 7 wherein the antibody reagent is labeled with a detectable chemical group selected from an enzymatically active group, a fluorescer, a chromophore, a luminescer, a specifically bindable ligand, or a radioisotope.

**Patentansprüche**

1. Verfahren zur Bestimmung einer bestimmten Polynukleotidsequenz in einem Testmedium, das einzelsträngige Nukleinsäuren enthält, umfassend die Stufen: Kombinieren des Testmediums mit einer Sonde, die mindestens eine einzelsträngige Basensequenz umfaßt, die im wesentlichen zu der zu bestimmenden Sequenz komplementär ist, unter Bedingungen, die günstig sind für die Hybridisierung zwischen der zu bestimmenden Sequenz und der komplementären Sondensequenz, wobei die komplementäre Sondensequenz im wesentlichen aus RNA besteht, wenn die zu bestimmende Sequenz RNA oder DNA ist, oder im wesentlichen aus DNA oder RNA besteht, wenn die zu bestimmende Sequenz RNA ist, und Bestimmung der hybridisierten Sonde durch Bindung eines Antikörperreagens, das zur Bindung von DNA/RNA- oder RNA/RNA-Duplexstrukturen, die zwischen der zu bestimmenden Sequenz und der komplementären Sondensequenz gebildet werden, und Bestimmung des Antikörperreagens, das an solche Duplex-Strukturen gebunden wird, dadurch gekennzeichnet, daß die Sonde eine spezifische Bindungsstelle umfaßt, und daß vor der Durchführung der Bestimmung der hybridisierten Sonde eine immobilisierte Form eines Bindungspartners für eine solche spezifische Bindungsstelle mit der Sonde in Kontakt gebracht wird, um die Sonde zu immobilisieren.

2. Verfahren nach Anspruch 1, dadurch gekennzeichnet, daß die Sonde in einer löslichen Form ist, wenn sie mit dem Testmedium in Kontakt gebracht wird, und, wenn sie mit der zu bestimmenden Sequenz hybridisiert wird durch Kontakt mit einem Adsorbens, das doppelsträngige Nukleinsäuren adsorbiert, immobilisierbar ist.

3. Verfahren nach einem der Ansprüche 1 und 2, dadurch gekennzeichnet, daß das Antikörperreagens mit einer bestimmbaren chemischen Gruppe markiert ist, ausgewählt aus einer enzymatisch aktiven Gruppe, einer fluoreszierenden Gruppe, einem Chromophor, einer lumineszierenden Gruppe, einem spezifisch bindbaren Liganden, oder einem Radioisotop.

4. Verfahren nach Anspruch 1, dadurch gekennzeichnet, daß das Testmedium eine biologische Probe umfaßt, die Bedingungen unterworfen wurde, um die darin vorhandenen Nukleinsäuren freizusetzen und zu denaturieren.

5. Reagenssystem zur Bestimmung einer bestimmten Polynukleotidsequenz in einem Testmedium durch Nukleinsäurehybridisierung, umfassend
   (i) eine Polynukleotidsonde, die eine spezifische Bindungsstelle umfaßt, und die mindestens eine einzelsträngige Basensequenz besitzt, die im wesentlichen komplementär zu der zu bestimmenden Sequenz ist, wobei die komplementäre Sondensequenz im wesentlichen aus RNA besteht, wenn die zu bestimmende Sequenz RNA oder DNA ist, oder im wesentlichen aus DNA oder RNA besteht, wenn die zu bestimmende Sequenz RNA ist, und
   (ii) eine immobilisierte Form eines Bindungspartners für die von der Sonde umfaßte spezifische Bindungsstelle, und
   (iii) ein Antikörperreagens, das dazu fähig ist, an DNA/RNA- oder RNA/RNA-Duplex-Strukturen, die zwischen der zu bestimmenden Sequenz und der komplementären Sondensequenz gebildet werden, zu binden, dadurch gekennzeichnet, daß die Sonde in einer immobilisierbaren Form ist.

6. Reagenssystem nach Anspruch 5, dadurch gekennzeichnet, daß das Antikörperreagens mit einer bestimmbaren chemischen Gruppe markiert ist, ausgewählt aus einer enzymatisch aktiven Gruppe, einer fluoreszierenden Gruppe, einem Chromophor, eines lumineszierenden Gruppe, einem spezifisch bindbaren Liganden, oder einem Radioisotop.

## Revendications

1. Une méthode de détermination d'une séquence particulière de polynucléotides dans un milieu de test contenant des acides nucléiques à un seul brin, comprenant les étapes de combinaison du milieu de test avec un échantillon comprenant au moins une séquence de bases à un seul brin qui est essentiellement complémentaire à la séquence à déterminer dans des conditions favorables à l'hybridation entre la séquence à déterminer et la séquence de l'échantillon complémentaire, la séquence de l'échantillon complémentaire étant essentiellement composée d'ARN si la séquence à déterminer est l'ARN ou l'ADN ou étant essentiellement composée d'ADN ou d'ARN si la séquence à déterminer est l'ARN et de détection de l'échantillon hybridé par liaison d'un réactif anticorps capable de se lier à des duplex ADN/ARN ou ARN/ARN formés entre la séquence à déterminer et la séquence de l'échantillon complémentaire et de détermination du réactif anticorps qui se lie à de tels duplex, caractérisée en ce que l'échantillon comprend un site de liaison spécifique et, avant d'effectuer ladite détection de l'échantillon hybridé, une forme immobilisée d'un partenaire de liaison pour un tel site de liaison spécifique entre en contact avec l'échantillon afin d'immobiliser ledit échantillon.

2. La méthode de la revendication 1 dans laquelle l'échantillon est sous forme soluble lorsqu'il entre en contact avec le milieu de test et est immobilisable une fois hybridé à la séquence à déterminer par contact avec un adsorbant qui adsorbe les acides nucléiques à double brin.

3. La méthode selon une des revendications 1 et 2 dans laquelle le réactif anticorps est marqué avec un radical chimique détectable choisi parmi les radicaux enzymatiquement actifs, les substances fluorescentes, les chromophores, les substances luminescentes, les ligands à liaison spécifique ou les radio-isotopes.

4. La méthode de la revendication 1 dans laquelle le milieu de test comprend un échantillon biologique qui a été soumis à des conditions de libération et de dénaturation des acides nucléiques qu'il contient.

5. Un système réactif de détection d'une séquence de polynucléotides particulière dans un milieu de test par hybridation des acides nucléiques comprenant (1) un échantillon de polynucléotides qui comprend un site de liaison spécifique et a, au moins, une séquence de bases à un seul brin qui est essentiellement complémentaire à la séquence à déterminer, la séquence d'échantillon complémentaire étant essentiellement composée d'ARN si la séquence à déterminer est l'ARN ou l'ADN ou étant essentiellement composée d'ARN ou d'ADN si la séquence à déterminer est l'ARN et (ii) une forme immobilisée d'un partenaire de liaison pour ledit site de liaison spécifique compris dans ledit échantillon et (iii) un réactif anticorps capable de se lier à des duplex ADN/ARN ou ARN/ARN formés entre la séquence à déterminer et la séquence d'échantillon complémentaire, caractérisé en ce que l'échantillon se trouve sour une forme immobilisable.

6. Le système réactif de la revendication 6 ou 7 dans lequel le réactif anticorps est marqué avec un radical chimique détectable choisi parmi les radicaux enzymatiquement actifs, les substances fluorescentes, les chromophores, les substances luminescentes, les ligand liables spécifiquement ou les radio-isotopes.

———————————— (S)    SINGLE STRANDED
                    SAMPLE NUCLEIC ACIDS

        +

———————————— (P)    BIOTIN - MODIFIED PROBE
    Bio

    1. HYBRIDIZE

    2.    ADD
        IMMOBILIZED
          AVIDIN

                        ———————————— (S)
                        ———————————— (P)
                            Bio
                           ( Av )
                           ////

        ADD  LABELED
          ANTIBODY

    *         *
  ( Ab )   ( Ab )
——————————————————— (S)    MEASURE LABEL
——————————————————— (P)    ON SOLID SUPPORT
        Bio
       ( Av )
       ////

                FIG. 1

                  11